# EUROPÄISCHE PATENTSCHRIFT

(11) **EP 2 364 646 B1**
(45) Veröffentlichungstag und Bekanntmachung des Hinweises auf die Patenterteilung: **26.09.2012**
(21) Anmeldenummer: 11154032.4
(22) Anmeldetag: 10.02.2011
(51) Int. Cl.: A61B 5/12, H04R 25/00

(54) **Hörtestverfahren**
Hearing test method
Procédé de test auditif

(30) Priorität: 09.03.2010 DE 102010010764
(43) Veröffentlichungstag der Anmeldung: 14.09.2011
(73) Patentinhaber: Siemens Medical Instruments Pte. Ltd., Singapore 139959 (SG)
(72) Erfinder: Chalupper, Josef, 85307, Paunzhausen (DE); Krämer, Beate Anna Maria, 90768, Fürth (DE)
(74) Vertreter: Maier, Daniel Oliver

(56) Entgegenhaltungen:
- US-A1- 2004 049 125
- STECKER G C ET AL: "Perceptual training improves syllable identification in new and experienced hearing aid users", JOURNAL OF REHABILITATION RESEARCH AND DEVELOPMENT 2006 REHABILITATION RESEARCH AND DEVELOPMENT SERVICE USA, Bd. 43, Nr. 4, 2006, Seiten 537-551, XP002630692, DOI: DOI:10.1682/JRRD.2005.11.0171
- FULLGRABE C ET AL: "Masking release for consonant features in temporally fluctuating background noise", HEARING RESEARCH, ELSEVIER SCIENCE PUBLISHERS, AMSTERDAM, NL, Bd. 211, Nr. 1-2, 1. Januar 2006 (2006-01-01), Seiten 74-84, XP025083407, ISSN: 0378-5955, DOI: DOI:10.1016/J.HEARES.2005.09.001 [gefunden am 2006-01-01]
- RICKETTS T ET AL: "Comparison of performance across three directional hearing aids.", JOURNAL OF THE AMERICAN ACADEMY OF AUDIOLOGY APR 1999 LNKD- PUBMED:10941709, Bd. 10, Nr. 4, April 1999 (1999-04), Seiten 180-189, XP002630693, ISSN: 1050-0545

## Beschreibung

Zum Test des Hörvermögens einer Person (Testperson) ist eine Vielzahl an Hörtestverfahren bekannt. Bei einem einfach zu realisierenden Hörtest wird der Person ein Ton einer bestimmten Frequenz dargeboten, zunächst sehr leise und mit der Zeit zunehmender Lautstärke. Die Person gibt ein Zeichen, sobald sie den Ton wahrnehmen kann, beispielsweise durch Betätigen eines Schalters. Dieser Test wird für verschiedene Frequenzen aus dem hörbaren Frequenzbereich wiederholt, so dass eine Aussage darüber möglich ist, ob die Person bei bestimmten oder bei allen gemessenen Frequenzen einen Hörverlust gegenüber einem Normalhörenden aufweist und gegebenenfalls lässt sich anhand der zur Wahrnehmung der jeweiligen Töne erforderlichen Lautstärke bereits eine Einschätzung über die Schwere eines Hörverlustes abgeben. Derartige Hörtests in Bezug auf die Hörschwelle bei unterschiedlichen Frequenzen (Reinton-Tests) werden bereits seit vielen Jahren über das Internet angeboten und können daher von interessierten Personen allein und bequem von zuhause aus durchgeführt werden. Nachteilig ist, dass eine relativ genaue Kalibrierung des von der Testperson verwendeten Lautsprechers bzw. Kopfhörers erforderlich ist.

Ein einfaches handhaltbares Hörtestgerät zur Durchführung eines Reinton-Hörtests ist beispielsweise aus der Druckschrift EP 1 813 190 A1 bekannt.

Weiterhin ist eine Vielzahl an Hörtests bekannt, mit denen eine Aussage über die Fähigkeit einer Person, Sprache zu verstehen, getroffen werden kann. Beispielhaft wird hierbei auf die vom Hörzentrum Oldenburg entwickelten Sprachverständlichkeitstests (z.B. Oldenburger Satztest) verwiesen.

Mitunter werden auch einfache Sprachverständlichkeitstests über das Internet angeboten, bei denen beispielsweise einfache Wörter (z.B. Apfel, Ball, Haus, eins, zwei, drei etc) vorgesprochen werden und eine Testperson z.B. auf einem Display die entsprechenden Wörter oder Symbole zur Auswahl dargeboten bekommt.

Bei der Durchführung der genannten Hörtests ist es ebenfalls bekannt, neben oder zusätzlich zu der Veränderung der Lautstärke der dargebotenen akustischen Signale bei der Darbietung auch Störsignale zu überlagern. Dabei kann bei der Durchführung eines entsprechenden Hörtests auch das Signal-Rausch-Verhältnis (SNR - Signal Noise Ratio) verändert werden. Vielen schwerhörigen Personen fällt es nämlich besonders schwer, Sprache bei gleichzeitigem Störlärm zu verstehen.

Aus der Veröffentlichung von Christian Lorenzi, Gaëtan Gilbert, Héloïse Carn, Stephane Garnier, Brian C. J. Moore: *"*Speech perception problems of the hearing impaired reflect inability to use temporal fine structure"; PNAS December 5, 2006, vol. 103, no. 49, 18869, und aus der Veröffentlichung "Masking release for consonant features in temporally fluctuating background noise"; Hearing Research, Bd. 211, Nr. 1-2 von Christian Füllgrabe et al. ist jeweils ein Sprachverständlichkeitstest bekannt, bei dem die Probanden sinnlose Silben, die sich beispielsweise jeweils aus einer Folge Vokal-Konsonant-Vokal zusammensetzten, erkennen sollen. Die Silben werden unter anderem auch zusammen mit überlagertem, fluktuierendem Störgeräusch dargeboten.

Aufgabe der vorliegenden Erfindung ist es, einen Hörtest anzubieten, der einfach und automatisch ausführbar ist und insbesondere auch über das Internet ausgeführt werden kann.

Diese Aufgabe wird durch ein Verfahren zum Test des Hörvermögens einer Person mit den Verfahrensschritten gemäß Patentanspruch 1 gelöst.

Vorteilhaft wird zur Durchführung des Hörtestverfahrens gemäß der Erfindung eine Hörtestanwendung auf einem über ein Netzwerk, insbesondere ein Computernetzwerk wie das Internet, zugänglichen Computer bereitgestellt. Die Person (Testperson) kann dann mit einem persönlichen Computer (PC, Notebook, PDA, Smartphone etc) eine Verbindung zu dem Computer herstellen, beispielsweise über das Internet und/oder ein Mobilfunknetz, und die Hörtestanwendung starten. Das Hörtestverfahren kann somit jederzeit automatisch und insbesondere ohne Anwesenheit von Fachpersonal wie eines HNO-Arztes, Audiologen etc durchgeführt werden.

Für die Beurteilung, ob ein Hörverlust vorliegt, erhält man mit der Darbietung von Sprachsignalen eine zuverlässigere Aussage im Vergleich zur Reintonaudiometrie, bei der lediglich Sinustöne dargeboten werden. Damit der Hörtest in unveränderter Form international, insbesondere sprachenunabhängig, durchführbar ist, umfasst das dargebotene Sprachsignal vorzugsweise sinnlose Silben, sog. Logatome. Diese können z.B. aus Vokal-Konsonant-Vokal- (aba, aca, ada,...) oder Konsonant-Vokal-Konsonant- (bab, beb, bib, ...) Folgen bestehen. Gegebenenfalls sind diese sinnlosen Silben noch dahingehend zu prüfen und nur solche auszuwählen, die in allen wichtigen Sprachen keinen Sinn ergeben. Die dargebotenen Silben resultieren vorzugsweise aus Sprachaufnahmen. Sie können aber auch synthetisch erzeugt werden.

Eine dargebotene Silbe (z.B. aba) wird schließlich zusammen mit anderen ähnlich klingenden Silben (z.B. ada, aga, aha, aka, ...) auf einer grafischen Benutzeroberfläche der Testperson dargestellt. Diese wählt aus den dargestellten Silben diejenige aus, z.B. durch ein Zeigerinstrument, die sie meint, gehört zu haben. Alternativ kann die von der Testperson erkannte Silbe von dieser auch nachgesprochen und durch eine Spracherkennung erfasst werden.

Schließlich erfolgt eine Auswertung der getroffenen Auswahl. Im einfachsten Fall wird dadurch die Auswahl als richtig oder falsch gekennzeichnet.

Der Test wird unter erneuter Auswahl sinnloser Silben wiederholt, solange, bis ein Abbruchkriterium erreicht ist.

Bei einer bevorzugten Ausführungsform der Erfindung wird dem dargebotenen Sprachsignal ein Störsignal, insbesondere Rauschen, überlagert. Bei der Darbietung von Sprachsignalen in Störlärm spielt nämlich die Kalibrierung des persönlichen Computers der Testperson eine untergeordnete Rolle. Die Testsignale müssen dann lediglich in einer Lautstärke dargeboten werden, mit der sie gut wahrnehmbar sind. Der Hörtest ist somit weitgehend unabhängig von der Art und Qualität des persönlichen Computers der Testperson. Die Sensitivität des Hörtest ergibt sich aus einer Variation des Signal-Rausch-Verhältnisses (SNR - Signal-Noise-Ratio). Dabei startet der Test zunächst mit keinem oder einem geringen Rauschanteil und der Rauschanteil wird dann relativ zu dem Sprachanteil mit jedem Durchlauf zunehmend vergrößert, solange, bis die Testperson das Sprachsignal nicht mehr richtig erkennt. Der Test endet bei einer möglichen Variante der Erfindung dann an dieser Stelle und ein Ergebnis des Tests ist das niedrigste Signal-Rausch-Verhältnis, das noch richtig erkannt wurde. Bei einer anderen Variante wird der Test mit dem Signal-Rausch-Verhältnis, das erstmals nicht mehr richtig erkannt wurde, wenigstens einmal wiederholt um festzustellen, ob ein reproduzierbares Testergebnis vorliegt. Gegebenenfalls wird der Test anschließend mit niedrigerem SNR fortgesetzt oder aber beendet, wenn auch die Wiederholung zu einem negativen Ergebnis geführt hat. Weiterhin kann bei einer Variante der Erfindung auch das niedrigste Signal-Rausch-Verhältnis, das noch fehlerfrei erkannt wurde, gegebenenfalls nochmals überprüft werden.

Das Verhältnis zwischen Sprachsignal und Störsignal, bei dem die Testperson das Sprachsignal nicht mehr erkennt, erlaubt dann eine zuverlässige Aussage darüber, ob eine Hörminderung vorliegt. Die Einstufung, ob eine Hörminderung vorliegt, ergibt sich insbesondere aus einem Vergleich des von der Testperson erreichten Ergebnisses mit einem für Normalhörende typischen Wertebereich. Fällt das von der Testperson erreichte Ergebnis in diesen Wertebereich, so wird diese als "Normalhörend" eingestuft. Vorzugsweise ergeht automatisch eine entsprechende Mitteilung an die Testperson. Konnte der Wertebereich Normalhörender nicht erreicht werden, so wird die Testperson als "Schwerhörig" eingestuft. Auch hierbei ergeht vorzugsweise eine entsprechende Mitteilung an die Testperson, ggf. unter Angabe weiterer Information, z.B. über eine mögliche Einstufung der Schwerhörigkeit (leicht, mittel, schwer), Beratungsmöglichkeiten etc.

Eine weitere Verbesserung der Zuverlässigkeit bzw. Sensitivität des Hörtests kann dadurch erreicht werden, dass als Störsignal fluktuierendes Rauschen, insbesondere amplitudenmoduliertes Rauschen mit einer vorzugsweise zufälligen Modulation verwendet wird. Durch die Verwendung eines modulierten Störgeräusches wird eine hohe Sensitivität für eine Hörminderung erreicht, da insbesondere bei einer Innenohrschwerhörigkeit die Schwelle bei modulierten Schallen gegenüber Normalhörenden deutlich angehoben ist.

Besonders zuverlässige und gute Ergebnisse ergeben sich weiterhin dann, wenn das Sprachsignal jeweils im Bereich eines Minimums des fluktuierenden Störsignals dargeboten wird.

Die Erfindung wird nachfolgend anhand von Ausführungsbeispielen näher erläutert. Dabei zeigen:
Figur 1 ein Ablaufdiagramm zur Ausführung eines erfindungsgemäßen Hörtestverfahrens und
Figur 2 die Durchführung eines Hörtests bei einer schwerhörigen und einer normalhörenden Testperson.

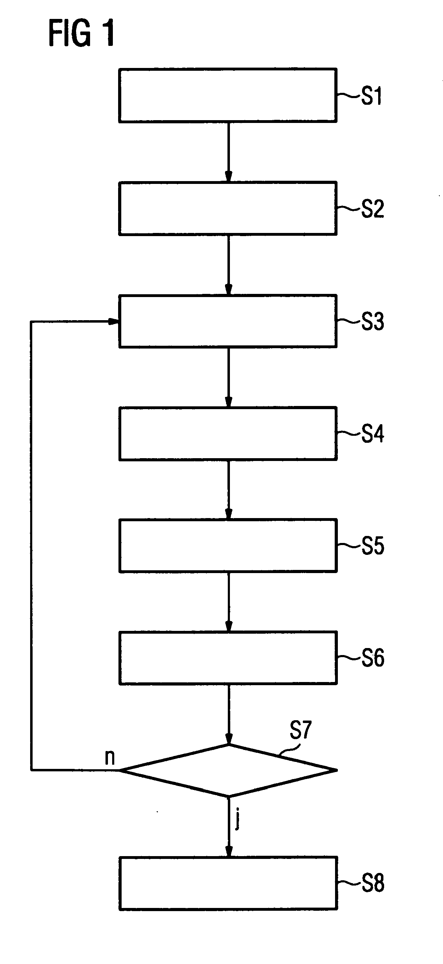

Bei der Durchführung eines beispielhaften Hörtestverfahrens gemäß Figur 1 wird in einem ersten Verfahrensschritt S1 eine Hörtestanwendung zur Ausführung des erfindungsgemäßen Verfahrens auf einem über ein Netzwerk, insbesondere das Internet, zugänglichen Computer bereitgestellt.

In einem zweiten Verfahrensschritt S2 stellt eine Person (Testperson) eine Verbindung zwischen dem Computer mit der bereitgestellten Hörtestanwendung und ihrem persönlichen Computer (PC, Notebook, PDA, Smarphone etc) her und startet, vorzugsweise mittels einer grafischen Benutzeroberfläche, die Hörtestanwendung. Die Bedienung erfolgt bevorzugt mittels Tastatur und/oder Computermaus. Es sind jedoch auch andere Steuerverfahren, z.B. Sprachsteuerung, möglich. Vorzugsweise wird der Hörtest durch Betätigung eines "Start-Buttons" gestartet.

Gegebenfalls erfolgt nun in einem weiteren Verfahrensschritt zunächst eine Abfrage bzw. ein Test, ob ein Kopfhörer angeschlossen oder ein Lautsprecher eingeschaltet ist und ob die eingestellte Lautstärke angenehm ist.

Im nachfolgenden Verfahrensschritt S3 erfolgt die automatische Auswahl und Darbietung eines akustischen Sprachsignals in Form einer sinnlosen Silbe aus einer Vielzahl möglicher sinnloser Silben, die nach Möglichkeit so vorausgewählt wurden, dass sie international verwendbar (international sinnlos) sind.

In einem Verfahrensschritt S4 wird die dargebotene Silbe zusammen mit anderen, vorzugsweise ähnlichen Silben auf einer grafischen Benutzeroberfläche des persönlichen Computers der Testperson dargestellt. Vorzugsweise sind die Silben in der Form Vokal-Konsonant-Vokal aufgebaut und die ähnlichen dargebotenen Silben unterscheiden sich vorzugsweise nur im Konsonanten.

Im nachfolgenden Verfahrensschritt S5 wählt die Testperson, vorzugsweise mit einem Zeigergerät (Computermaus) auf der grafischen Bedienoberfläche, die dargestellte sinnlose Silbe aus den dargestellten Silben aus, die sie meint gehört zu haben. Gegebenfalls können auch Buttons für "Test wiederholen" oder "nicht verstanden" zur Betätigung angeboten werden. Schließlich erfolgt in einem nachfolgenden Verfahrensschritt S6 eine automatische Auswertung der von der Testperson getroffenen Auswahl. Insbesondere wird erfasst, ob die Auswahl der tatsächlich dargebotenen Silbe entspricht. Weiterhin wird in einem Verfahrensschritt S7 entschieden, ob der Test durch einen Sprung zu dem Verfahrensschritt S3 durch die Auswahl einer neuen Silbe fortgeführt oder in einem Verfahrensschritt S8 beendet werden soll. Der Test wird insbesondere dann beendet, wenn ein Abbruchkriterium erreicht ist, z.B. ein eindeutiges Ergebnis über das Hörvermögen der Testperson vorliegt oder eine bestimmte Anzahl an Durchläufen (z.B. 8) erfolgt ist. Liegt kein Abbruchkriterium vor, so wird der Hörtest bei Verfahrensschritt S3 fortgesetzt und erneut eine Silbe ausgewählt und dargeboten.

Um eine Aussage über das Hörvermögen der Testperson treffen zu können wird dem in Verfahrensschritt S3 dargebotenen Sprachsignal ein Störsignal überlagert. Bei diesem kann es sich um ein Rauschsignal (z.B. weißes Rauschen) handeln. Das dargebotenen Sprachsignal und/oder das Störsignal wird dann mit jedem Durchlauf des Tests derart variiert, dass sich das Verhältnis der Signalpegel zwischen dem Sprachsignal und dem Störsignal (SNR) ändert.

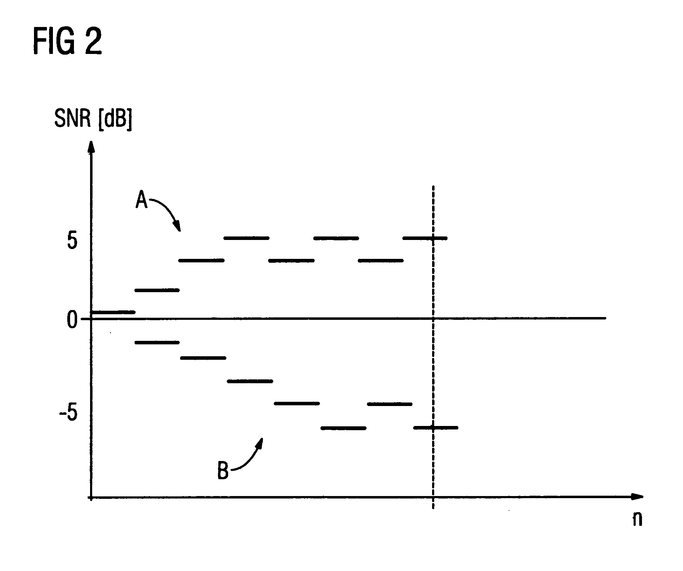

Eine mögliche Abfolge bei der Variation des mittleren Signalpegels des Störsignals bei gleichbleibendem Signalpegel der nacheinander dargebotenen Sprachsignale und damit eine Variation des Signal-Rausch-Verhältnisses (SNR) zeigt Figur 2. In das Schaubild sind sowohl der Testverlauf für eine normalhörende Testperson (unterer Kurvenverlauf "B") als auch für eine schwerhörige Testperson (oberer Kurvenverlauf "A") eingezeichnet.

Der Test startet im Ausführungsbeispiel mit einem SNR von ca. 0 dB, d.h. der mittlere Signalpegel des Sprachsignals und der des Störsignals sind in etwa gleich. Erkennt die Testperson die so dargebotene Silbe korrekt, so verläuft der weitere Test gemäß dem unteren Kurvenverlauf B. Dabei wird das Signal-Rausch-Verhältnis dadurch herabgesetzt, dass entweder bei gleichbleibendem mittlerem Signalpegel des Rauschsignals der Signalpegel des Sprachsignals gegenüber dem vorherigen Testdurchlauf gesenkt wird oder dass der Signalpegel der nacheinander dargebotenen Sprachsignale beibehalten und der Störsignalpegel nach jedem Durchlauf angehoben wird. Das Signal-Rausch-Verhältnis wird dann mit jedem Durchlauf stufenweise so lange herabgesetzt, bis die Testperson die zuletzt gesprochene Silbe nicht mehr richtig erkennt. Dann wird entweder der Test beendet oder - zur Kontrolle - die zuletzt erfolgte Verschlechterung des SNR wieder rückgängig gemacht und erneut eine Silbe dargeboten um zu testen, ob diese wieder richtig erkannt wird und damit die Schwelle der Silbenerkennung unter Störgeräuscheinfluss korrekt erfasst wurde.

Erkennt die Testperson die zuerst dargebotene Silbe nicht richtig, so verläuft der weitere Test beispielsweise gemäß dem oberen Kurvenverlauf A von Figur 2. Dabei wird das Sprachsignal gegenüber dem Störsignal stufenweise solange angehoben, bis die Testperson die zuletzt gesprochene Silbe richtig erkennt. Auch hier kann zur Kontrolle die letzte Anhebung des SNR wieder rückgängig gemacht werden, um die Schwelle, bei der die dargebotenen Silben zuverlässig erkannt werden, exakt auszutesten. Im Ausführungsbeispiel gemäß Figur 2 endet der Hörtest jeweils nach 8 Durchläufen automatisch.

Bei einer bevorzugten Ausführungsform der Erfindung ist der Betrag, um den das Stör-Rausch-Verhältnis zwischen zwei Testdurchläufen verbessert bzw. verschlechtert wird, variabel. Der Test beginnt dabei zunächst mit verhältnismäßig großen Stufen, solange, bis erstmalig eine Silbe nicht mehr richtig erkannt wurde bzw. bis erstmalig eine Silbe richtig erkannt wurde. Dann wird um den Wert des zuletzt eingestellten SNR mit kleineren Stufen wie oben beschrieben fortgefahren, um die Schwelle, bei der die dargebotenen Silben nicht mehr zuverlässig erfasst werden, möglichst eng einzugrenzen.

## Patentansprüche

1. Verfahren zum Test des Hörvermögens einer Person durch:
a) Bereitstellen einer Hörtestanwendung auf einem über ein Netzwerk zugänglichen Computer,
b) Aufruf der Hörtestanwendung durch die Person über das Netzwerk mittels eines persönlichen Computers der Person,
c) Auswahl und Darbietung eines akustischen Sprachsignals in Form wenigstens einer sinnlosen Silbe,
d) Darstellung der sinnlosen Silbe und einer Anzahl weiterer sinnlosen Silben auf einer grafischen Benutzeroberfläche des persönlichen Computers,
e) Auswahl einer gehörten sinnlosen Silbe aus den dargestellten sinnlosen Silben durch den Benutzer,
f) Auswertung der getroffenen Auswahl,
g) Wiederholen der Verfahrensschritte c) bis f), bis ein Abbruchkriterium erreicht ist,
wobei dem dargebotenen Sprachsignal ein Störsignal, insbesondere Rauschen, überlagert wird,
wobei ein fluktuierendes Störsignal überlagert wird, **gekennzeichnet dadurch, dass** das Sprachsignal jeweils im Bereich eines Minimums des fluktuierenden Störgeräusches dargeboten wird.

2. Verfahren nach Anspruch 1, wobei die Auswahl und/oder Darbietung des akustischen Sprachsignals und/oder des Störsignals zur Wiederholung der Verfahrensschritte c) bis f) in Abhängigkeit der Auswertung der von der Person getroffenen Auswahl bezüglich wenigstens eines zuvor erfolgten Durchlaufes der Verfahrensschritte c) bis f) erfolgt.

3. Verfahren nach Anspruch 2, wobei das Signal-RauschVerhältnis zwischen dem Sprachsignal und dem Störsignal angepasst wird.

4. Verfahren nach Anspruch 3, wobei das Signal-RauschVerhältnis in Stufen variabler Höhe angepasst wird.

## Claims

1. Method for testing the hearing of a person, by:
a) providing a hearing-test application on a network-accessible computer,
b) calling the hearing-test application by the person via the network by means of a personal computer of the person,
c) selecting and presenting an acoustic speech signal in the form of at least one meaningless syllable,
d) presenting the meaningless syllable and a number of additional meaningless syllables on a graphical user interface of the personal computer,
e) the user selecting a heard meaningless syllable from the displayed meaningless syllables,
f) evaluating the selection that was made,
g) repeating method steps c) to f) until an interrupt criterion is satisfied,
wherein an interference signal, more particularly noise, is superposed onto the presented speech signal,
wherein a fluctuating interference signal is superposed, **characterized in that**
the speech signal is in each case presented in the region of a minimum of the fluctuating interference noise.

2. Method according to Claim 1, wherein the acoustic speech signal and/or the interference signal are/is selected and/or presented for repeating method steps c) to f), depending on the evaluation of the selection made by the person in respect of at least one previous run-through of method steps c) to f).

3. Method according to Claim 2, wherein the signal-to-noise ratio between the speech signal and the interference signal is modified.

4. Method according to Claim 3, wherein the signal-to-noise ratio is modified by levels with variable heights.

## Revendications

1. Procédé de test du pouvoir auditif d'une personne, dans lequel :
a) on prépare une application de test auditif sur un ordinateur accessible par un réseau,
b) on appelle l'application de test auditif par la personne par le réseau au moyen d'un ordinateur personnel de la personne,
c) on choisit et on présente un signal vocal acoustique sous la forme d'au moins une syllabe dénuée de sens,
d) on présente la syllabe dénuée de sens et un certain nombre d'autres syllabes dénuées de sens sur une surface graphique d'utilisateur de l'ordinateur personnel,
e) on fait choisir par l'utilisateur une syllabe entendue et dénuée de sens parmi les syllabes représentées et dénuées de sens,
f) on exploite le choix effectué,
g) on répète les stades c) à f) du procédé jusqu'à obtention d'un critère d'interruption,
dans lequel on superpose au signal vocal représenté un signal parasite, notamment du bruit,
dans lequel on superpose un signal parasite fluctuant, **caractérisé en ce que** le signal vocal est représenté respectivement dans la zone d'un minimum du bruit parasite fluctuant.

2. Procédé suivant la revendication 1, dans lequel le choix et/ou la représentation du signal vocal acoustique et/ou du signal parasite est effectué pour la répétition des stades c) à f) du procédé en fonction de l'exploitation du choix effectué par la personne en ce qui concerne au moins une passe effectuée auparavant des stades c) à f) du procédé.

3. Procédé suivant la revendication 2, dans lequel on adapte le rapport signal/bruit entre le signal vocal et le signal parasite.

4. Procédé suivant la revendication 3, dans lequel on adapte le rapport signal/bruit en paliers de hauteur variable.
